Europäisches Patentamt

European Patent Office

Office européen des brevets

(11) Publication number: **0 193 284**
**B1**

# EUROPEAN PATENT SPECIFICATION

(45) Date of publication of patent specification: **18.07.90**

(21) Application number: **86300600.3**

(22) Date of filing: **29.01.86**

(51) Int. Cl.⁵: **G 01 N 33/569,**
**G 01 N 33/577**

(54) A method for detecting HTLV-III neutralizing antibodies in sera.

(30) Priority: **05.02.85 US 698588**

(43) Date of publication of application:
**03.09.86 Bulletin 86/36**

(45) Publication of the grant of the patent:
**18.07.90 Bulletin 90/29**

(84) Designated Contracting States:
**AT BE CH DE FR GB IT LI LU NL SE**

(56) References cited:
**WO-A-85/04903**

**BIOLOGICAL ABSTRACTS, vol. 80, no. 4, April
1985, no. 36659, Philadelphia, PA (US); K.NAGY
et al.: "Detection of receptors and neutralizing
antibodies of the human T cell leukemia virus by
pseudotype method"**

(73) Proprietor: **THE UNITED STATES OF AMERICA
as represented by the Secretary U.S.
Department of Commerce
National Technical Information Service Office of
Government Inventions and Patents 5285 Port
Royal Road
Springfield, Virginia 22161 (US)**

(72) Inventor: **Guroff, Marjorie Robert
6116 Tilden Lane
Rockville, MD 20852 (US)**
Inventor: **Gallo, Robert Charles
8513 Thornden Terrace
Bethesda, MD 20834 (US)**

(74) Representative: **Jump, Timothy John Simon
et al
F.J. Cleveland and Company 40-43 Chancery
Lane
London WC2A 1JQ (GB)**

Courier Press, Leamington Spa, England.

(56) References cited:
PROCEEDINGS OF THE NATIONAL ACADEMY
OF SCIENCES, MEDICAL SCIENCES, vol. 81, no.
8, May 1984, pages 2886-89, US; P. CLAPMAN et
al.: "Pseudotypes of human T-cell leukemia
virus types 1 and 2: neutralization by patients'
sera"

BIOLOGICAL ABSTRACTS, vol. 72, no. 2,
February 1981, no. 10890, Philadelphia, PA (US);
J.M.MILLER et al.: "Comparison of 4 serologic
tests for the detection of antibodies to bovine
leukemia virus"

SCIENCE, vol. 226, 26th October 1984, pages
451-453, Washington, DC; D.D. HO et al.:
"HTLV-III in the semen and blood of a healthy
homosexual man"

JOURNAL OF EXPERIMENTAL MEDICINE, vol.
159, April 1984, pages 1117-1131; T.J. PALKER
et al.: "Monoclonal antibodies against human T
cell leukemia-lymphoma virus (HTLV) p24
internal core protein"

## Description

Background

During the recent past in 1984 the virus HTLV-III emerged as the most probable causative agent of acquired immune deficiency syndrome (AIDS) illness (see Gallo, et al, in the Material Information Disclosure, post 1, 2, 3). Also when an H9 cell is suitably infected with the HTLV-III virus and cultivated, an immortalized product results. The HTLV p24 core antigen has been isolated and purified from the immortalized H9/HTLV-III cell line ATCC Accession No. CRL 8543.

Generalized process

In the present invention the natural antibodies in sera are assessed for their ability to neutralize HTLV-III infection. HTLV-III infection is preferably monitored by following expression of the viral core protein, p24, by means of a specific monoclonal antibody to HTLV-III p24.

Also in the present invention the effect is to use sera with accompanying antibodies within it to effectively neutralize an amount of virus. This antibody neutralization may be either in whole or in part and a quantitative estimate of neutralizing antibody titer may be made using the outlined procedures. Ths method is applicable to serum from any species and hence is useful for assessing potential vaccine preparations for effectiveness in eliciting an HTLV-III neutralizing antibody response. In the last step, when it is not possible to observe viral infection within three days, then the serum has neutralized the viral infectivity in toto. It is believed that the neutralizing antibody in the sera bind to the viral envelope glycoprotein which is responsible for the initial attachment of the virus to the receptors and, thus, blocks the infective action of the virus.

Also, the effort is made to utilize for a special purpose assays for HTLV-III dependent on antigen-antibody reaction and the presence of antibodies in sera of AIDS and related patients which neutralize viral antigen and are useful for protection.

Sketch I shows the process of the present invention.

Sketch I

Virus (HTLV-III)
+
Serum (with or without neutralizing antibodies) Binding of specific antibody to viral antigen Infect H9 cells and cultivate (3 day hold)

Assay for virus infectivity by monitoring expression of HTLV-III p24.

Material information disclosure

1)  Sarngadharan, et al, "Antibodies Reactive with Human T-lymphotropic Retroviruses (HTLV-III) in the Serum of Patients with AIDS" *Science*, 224:506—508, 1984.

2)  Safai, et al, "Seroepidemiological Studies of Human T-lymphotropic Retrovirus Type III in Acquired Immunodeficiency Syndrome", *Lancet*, i, 1438—1440, 1984.

3)  Gazzolo, et al, "Antibodies to HTLV-III in Haitian Immigrants in French Guiana", *New Engl. J. Med.*, 311:1252—1253, 1984.

4)  Clumeck, et al, "Seroepidemiological Studies of HTLV-III Antibody Prevalence Among Selected Groups Heterosexual Africans", to be presented at the International Conference on AIDS, Atlanta, April 14—17, 1985.

5)  Gonda, et al, "Sequence Homology and Morphologic Similarity of the AIDS virus, Human T-cell Lymphotropic Virus Type III (HTLV-III), and Visna Virus, Member of the Pathogenic Lentivirus Subfamily", *Science*, in press.

6)  Robert-Guroff, et al, "Detection of the Human T-cell Lymphoma Virus p19 in Cells of Some Patients With Cutaneous T-cell Lymphoma and Leukemia Using a Monoclonal Antibody, "*J. Exp. Med.*, 154:1957—1964, 1981.

None of the above references disclose the present method for detecting natural antibodies in sera which neutralize HTLV-III and protect therefor and measure the residual viral infectivity with a specific MAB such as anti-p24 HTLV-III (BT3 Biotech Research Labs, Veronese et al submitted).

The invention

In a first aspect of the invention there is provided a method for tha mesuring, *in vitro* the neutralization of HTLV-III virus infectivity, comprising:

(a) treating said virus with natural human antibodies in sera containing antibody to HTLV-III; and

(b) assaying residual viral infectivity to determine the measure of protection afforded by the natural antibodies.

In a second aspect of the invention there is provided a method for measuring *in vitro* the neutralization of HTLV-III virus infectivity, comprising:

(a) treating said virus with sera containing natural human antibodies;

(b) infecting and incubating the resulting culture with H9 cells; and

(c) assaying for residual infectivity with a monoclonal antibody specific for HTLV-III or HTLV-III p24.

In a last aspect of the invention there is provided a method for the *in vitro* assay of the protective effects induced by neutralizing HTLV-III antibodies on HTLV-III virus by addition of sera containing neutralizing antibodies, comprising measuring residual viral infectivity by addition of a monoclonal antibody specific for HTLV or p24 HTLV.

The isolation of the human T-cell leukemia (lymphotropic) virus type III (HTLV-III) from cells of numerous patients with the acquired immunodeficiency syndrom (AIDS) presented the first evidence that the virus was the etiologic agent of the disease. This conclusion has been strengthened by the results of many subsequent investigations including those of seroepidemiologic studies which showed the presence of HTLV-III specific antibodies in the serum of the vast majority of patients with AIDS and AIDS-related complex (ARC). In addition, viral specific antibodies have been found in the serum of every group of originally identified as a risk for AIDS, including homosexual males, hemophiliac recipients of factor VIII, intravenous drug users, and Haitians. More recent and wide-ranging serologic studies have identified additional populations exposed to the virus including heterosexual partners of AIDS or ARC patients and individuals from certain regions of Africa, especially Zaire and Rwanda, where AIDS as well as HTLV-III appear to be endemic.

While these sero-epidemiologic studies have provided many insights into the mode of transmission and extent of HTLV-III infection, there have been no reports concerning possible protective or therapeutic effects of HTLV-III specific antibodies in sero-positive individuals. Therefore, an investigation was conducted to determine if AIDS and ARC patients possess antibody activities capable of inhibiting viral infection. Such a natural defense mechanism enables an infected host to avoid cell to cell spread of the virus and, hence, progression of the disease may be retarded or prevented. In several animal retroviral systems, neutralizing antibodies have been described which bind to the viral envelope glycoprotein which is responsible for initial attachment of the virus to the target cell receptor (Steeves, R.A., et al., *J. Virol.,* 14:187—189, 1974). By blocking the binding of virus to this receptor, virus neutralizing antibodies may effectively inhibit viral infection. In the studies reported here, it was asked whether HTLV-III elicited specific neutralizing antibodies in AIDS or ARC patients and whether any protective effect of such antibodies could be demonstrated.

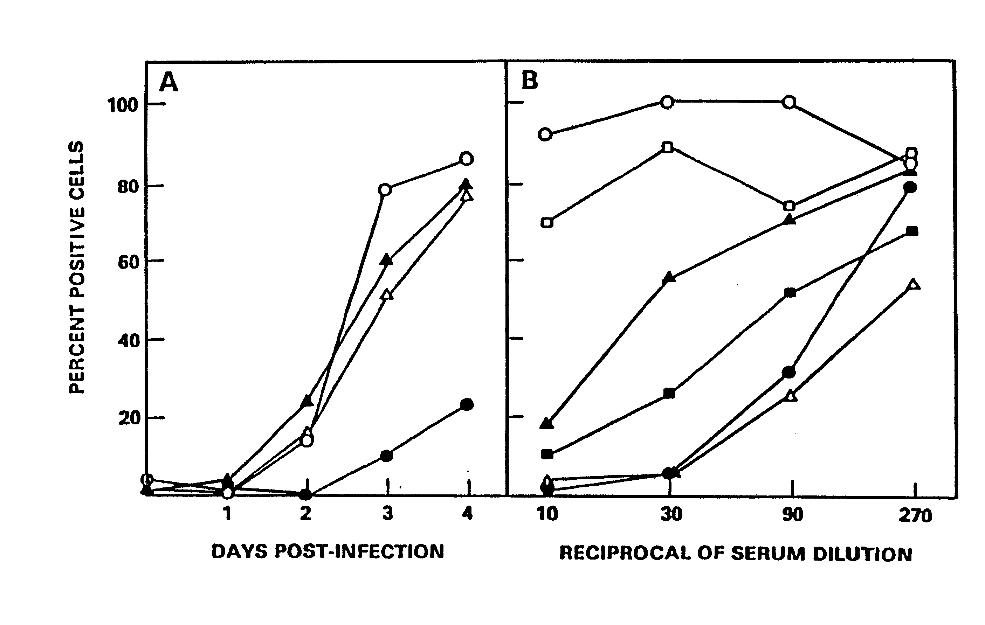

The H9 clone of the HT cell line (specific process and examples) was used as target for cell-free HTLV-III infection, and several sera were initially analyzed for virus neutralizing antibody activity. Infection of the H9 cells was assessed by monitoring the expression of HTLV-III p24 using a monoclonal antibody in an indirect immune fluorescence assay. Figure 1 illustrates the kinetics of infection of H9 cells with HTLV-III virus preincubated with sera positive or negative for virus neutralizing activity. By three days post-infection, approximately 80% of the H9 cells incubated with HTLV-III pretreated with serum of a healthy normal donor were infected as indicated by their expression of HTLV-III p24. In contrast, only 10% of H9 cells expressed HTLV-III p24 at day three when infected with virus pretreated with serum from a patient with ARC. That this inhibition of infection was mediated by a viral rather than a cellular antigen was shown by ready infection of H9 cells with HTLV-III following pre-treatment of the cells rather than the virus with the same sera (Figure 1a). The inhibitory activity of certain sera was not simply a non-specific effect of high serum concentrations because the activity was titratable. As illustrated by the several sera titrated in Figure 1b, sera possessing inhibitory activity were found in all categories of patients and healthy members of groups at risk for AIDS.

In order to confirm that the inhibitory activity detected was directed against a viral rather than a cellular antigen, sera were absorbed with preparations of cell-free virus or with infected or uninfected H9 cells. While absorption with cells had little effect, absorption with viral preparations substantially decreased titers of sera with inhibitory activity as shown in Table 1 below.

Natural antibodies capable of neutralizing HTLV-III infection of H9 cells were detected in 60% of adult AIDS patients and in 80% of adults with ARC, but in 0% of normal healthy heterosexual controls. Geometric mean antibody titers were two-fold higher in ARC patients compared to AIDS patients were even higher in 2 antibody positive healthy homosexuals. This finding suggests that virus neutralizing antibodies may exert some in vivo protective effect. The presence of these antibodies indicates an immunologic response to HTLV-III which may be utilized for therapeutic advantage. Also, the methodology employed in these studies can be directely useful in monitoring future vaccine approaches.

Therefore, having defined a system in which serum IgG could neutralize the infectivity of HTLV-III for H9 cells by binding to the virus, a number of human sera were analyzed for this antibody activity. The results are summarized in Table 2 below. It is clear that a high prevalence of patients with either AIDS or ARC possess virus neutralizing antibodies in contrast to healthy heterosexual individuals in which no such activity was demonstrated. While antibody titers ranged upwards of 500 for both patient groups, overall titers were low. However, it was observed that ARC patients possessed a two-fold higher geometric mean antibody titer compared to that of the AIDS patients studied. It was also seen that among healthy homosexuals for risk for development of AIDS, the geometric mean antibody titer, albeit determined on only 2 antibody positive individuals, was substantially higher than that of either of the two patient groups.

## EP 0 193 284 B1

This trend of higher titer with less or insigificant disease manifestations suggests a protective effect of the neutralizing antibodies.

HTLV-III neutralizing antibody activity was not detected in any normal healthy heterosexual individuals (Table 2). However, a barely detectable titer (of 13) was obtained in one of 4 serum samples from patients with acute mononucleosis. This result may suggest some weak cross-reactivity with viral antigen in sera possessing high levels of heterophilic antibodies.

In other retroviral systems, the major envelope glycoprotein is the target for neutralizing antibody. These naturally occurring virus neutralizing antibodies may be meaningful with regard to an *in vivo* protective effect.

The results of the present invention show a trend that individuals with less severe disease or those infected with HTLV-III but not yet manifesting clinical symptoms, possess higher neutralizing antibody titers. This suggests a human vaccine approach may be worthwhile. On the other hand, it is interesting to speculate that the role of neutralizing antibody in the overall biology of HTLV-III may be similar to that found in the visna virus system. Infection with visna virus, a nononcogenic retrovirus which causes a slowly progressive disease in sheep affecting primarily the lungs and central nervous system, is persistent. It has been shown that neutralizing antibodies elicited by the virus have a narrow range of specificity which cannot inhibit infection by mutant viruses which arise during the course of the disease. Thus, the neutralizing antibodies exert a selective pressure, leading to replication of non-neutralized mutant viruses. It is also of interest that "early sera" taken from relatively recently infected animals possess a more restricted neutralization range compared to "late sera" obtained from animals infected for more than three years. These "late sera" were able to neutralize a broader range of visna mutants including all ancestral strains. This is relevant to HTLV-III especially because of the demonstrated genomic variability from isolate to isolate, particularly in the viral envelope region and also because of the demonstrated relatedness of HTLV-III to visna virus (Gonda, et al, *Science*, in press).

The demonstration of HTLV-III neutralizing antibodies in sera of patients with AIDS and ARC and in healthy individuals infected with HTLV-III is a meaningful finding which demonstrates an immunologic response during the course of disease development which may be utilized for therapeutic advantage. It furthermore indicates that appropriate vaccine approaches may be effective in preventing viral infection from the outset. The methodology described here will be useful in monitoring these future procedures and will also be useful in additional basic investigations concerning the biology of HTLV-III infection. Further studies will determine whether the presence of virus neutralizing antibodies in patient sera have any prognostic value or will be indicative of appropriate treatment regimens.

Description of the figures

Sera from a normal healthy heterosexual (o) and from a patient with ARC (●) are compared in Figure 1A. In a parallel experiment, these same sera were preincubated with H9 cells for 1 h at 4°C. The cells were washed with PBS, incubated with the cell-free HTLV-III preparation, and cultured as in Example 2. Results of this treatment of the H9 cells with sera are represented for the normal, healthy heterosexual serum (△) and the serum of the patient with ARC (▲).

In Figure 1B representative titrations are shown for two sera negative for viral neutralizing antibody activity: a patient with AIDS (o) and a patient with ARC (□). Representative sera positive for virus neutralizing antibody were obtained from a pediatric AIDS case (●), a patient with ARC (■), a healthy homosexual (△), and an adult AIDS patient (▲). All values obtained were normalized to the level of infection attained in the presence of a standard antibody-negative serum.

5

## TABLE 1
### HTLV-III Neutralizing activity is a property of IgG and is directed against a viral antigen

| Serum samples | Patient diagnosis | Serum treatment (a) | HTLV-III Neutralizing antibody titer (b) |
|---|---|---|---|
| Experiments with purified IgG: | | | |
| 1 | ARC | None | 115 |
| | | Purification of IgG | 110 |
| 2 | AIDS | None | 34 |
| | | Purification of IgG | 40 |
| 3 | ARC | None | 60 |
| | | Purification of IgG | 76 |
| Absorption experiments: | | | |
| 4 | ARC | None | 135 |
| | | Absorbed with HTLV-III | <10 |
| | | Absorbed with H9 Cells | 120 |
| | | Absorbed with H9/HTLV-III | 90 |
| 5 | AIDS | None | 75 |
| | | Absorbed with HTLV-III | <10 |
| | | Absorbed with H9 Cells | 22 |
| | | Absorbed with H9/HTLV-III | 34 |
| 6 | ARC | None | >270 |
| | | Absorbed with HTLV-III | 50 |
| | | Absorbed with H9 Cells | >270 |
| | | Absorbed with H9/HTLV-III | >270 |

AIDS=acquired immunodeficiency syndrome
ARC=AIDS related complex

a) IgG was purified from 0.5 ml aliquots of human serum by absorption to protein A-Sepharose equilibrated in PBS. Following extensive washing of the columns with PBS, IgG was eluted with 0.1 M glycine-HCl, pH 2.8. The eluate was neutralized with 2M Tris-HCl, pH 8.0, dialyzed extensively against 10 mM ammonium bicarbonate, and lyophilized. The purified fractions were dissolved in 0.5 ml PBS, filter sterilized, and diluted in media for titration of neutralizing antibody activity as described in Example 2.

For virus absorption experiments, 62 ml of cell-free virus supernatant containing 2 to $5 \times 10^8$ virus particles/ml were pelleted as described in Example 2. The viral pellet was resuspended in 100 l of a 1:10 dilution of serum to be absorbed and incubated overnight at 4°C. The virus was again pelleted by centrifugation and the absorbed serum was saved for titration of virus neutralizing antibody activity. Sera were similarly absorbed on pellets of washed $10^7$ cells and titered.

b) Values for percent of HTLV-III p24-positive cells were normalized to the level of infection obtained in the presence of a standard negative serum treated similarly as the test serum. Antibody titers were then expressed as the reciprocal of the serum dilution at which virus infection was 60% of that obtained in the presence of this standard negative serum.

TABLE 2
HTLV-III Neutralizing antibody in AIDS and ARC patients and others at risk[a]

| Serum source | No. positive/ No. tested | Percent positive | Range of titer | Geometric mean titer |
|---|---|---|---|---|
| Adult AIDS patients | 21/35 | 60 | 10—520 | 44 |
| Pediatric AIDS patents | 9/9 | 33 | 80—180 | 117 |
| Adult ARC patients | 28/35 | 80 | 17—560 | 88 |
| Healthy homosexuals | 2/12 | 17 | 130—340 | 210 |
| Healthy heterosexuals | 0/20 | 0 | — | — |
| Heterosexual partners of AIDS patents[b] | 1/3 | 33 | 78 | — |
| Mothers of pediatric AIDS patents[c] | 0/2 | 0 | — | — |
| Siblings of AIDS patents[d] | 1/2 | 50 | 55 | — |
| Patients with acute mononucleosis | 1/4 | 25 | 13 | — |
| Patent with sarcoidosis | 0/1 | 0 | — | — |

AIDS=acquired immunodeficiency syndrome;
ARC=AIDS related complex

[a] All sera were screened for virus neutralizing antibody at a 1:10 dilution. Those sera possessing activity were further titered as described in Example 2. Antibody titer is defined in the footnote to Table 1.

[b] All 3 individuals were positive for HTLV-III antibodies by the ELISA and Western blot assays.

[c] Sera from these 2 foster mothers were negative for HTLV-III antibodies by the ELISA and Wester blot assays.

[d] The positive sibling was also antibody positive by the ELISA and Western blot assays.

Example 1

For virus absorption experiments, 62 ml of cell-free virus supernatant containing 2 to $5 \times 10^8$ virus particles/ml were pelleted as described in Example 2. The viral pellet was resuspended in 100 μl of a 1:10 dilution of serum to be absorbed and incubated overnight at 4°C. The virus was again pelleted by centrifugation and the absorbed serum was saved for titration of virus neutralizing antibody activity. Sera were similarly absorbed on pellets of washed $10^7$ cells and titered.

Values for percent of HTLV-III p24-positive cells were normalized to the level of infection obtained in the presence of a standard negative serum. Antibody titers were then expressed as the reciprocal of the serum dilution at which virus infection was 60% of that obtained in the presence of a standard negative serum.

Example 2

In the method for screening human sera for HTLV-III neutralizing antibodies, media containing 2 to $5 \times 10^8$ HTLV-III particles/ml were harvested from H9/HTLV-III cells. The amount of virus initially used was determined by titrating a virus preparation and selecting an amount for the assay which would achieve 50 to 80% of infected H9 cells by 3 days post infection. This in general required a substantial excess of virus particles per target cell. Cells were removed by low-speed centrifugation and the virus-containing supernatant was centrifuged for 3 hours at $32,000 \times g$. The viral pellets were resuspended in a total volume of 2.25 ml media (RPMI 1640 containing 20% fetal calf serum and penicillin/streptomycin). Uninfected H9 cells were washed in media and incubated for 20 minutes at room temperature in media containing 2 μg/ml polybrene. The cells were washed in media and resuspended at a concentration of $4 \times 10^6$/ml in media. Sera to be tested were heat inactivated at 56°C for 30 min and filter sterilized. For each assay 20 μl of virus suspension and 20 μl of a 1:10 dilution of serum was mixed and incubated in a well of a microtiter plate for 1 h at 4°C and then 15 min at room temperature. H9 cells (10 μl) were added to each well and incubation was continued for 1 h at 37°C. Aliquots (15 μl) of each mixture were plated into 200 μl media in duplicate wells of another microtiter plate. Cultures were incubated at 37°C in a 5% $CO_2$ incubator. After 3 days, cultures in individual wells were removed, washed 2 times with phosphate buffered saline (PBS) and once with PBS:water, 1:1. Cells were suspended in approximately 30 μl of the same solution and 5 to 10 μl aliquots

were spotted on 8-well toxoplasmosis slides for an indirect fixed-cell immune fluorescent assay using a monoclonal antibody to HTLV-III p24.

Sera exhibiting neutralizing antibody activity and a 1:10 dilution were subsequently serially diluted and the assay was repeated to determine antibody titer.

In the following claims and in the specification sera refers to sera containing a substantial quantity of anti-HTLV-III. This includes sera from adult and pediatric AIDS and ARC patients and healthy homosexual (see Table 2). The geometric titer ranges from about 44 to 210.

**Claims**

1. A method for the measuring, *in vitro* the neutralization of HTLV-III virus infectivity, comprising:
(a) treating said virus with natural human antibodies in sera containing antibody to HTLV-III; and
(b) assaying residual viral infectivity to determine the measure of protection afforded by the natural antibodies.

2. The method of Claim 1, wherein the assaying is carried out with the monoclonal antibody specific for HTLV-III.

3. The method of Claim 2, wherein the monoclonal antibody is anti-HTLV-III p24.

4. A method for measuring *in vitro* the neutralization of HTLV-III virus infectivity, comprising:
(a) treating said virus with sera containing natural human antibodies;
(b) infecting and incubating the resulting culture with H9 cells; and
(c) assaying for residual infectivity with a monoclonal antibody specific for HTLV-III or HTLV-III p24.

5. The method of Claim 4, wherein the monoclonal antibody is specific for HTLV-III p24.

6. The method of Claim 4, wherein the monoclonal antibody is specific for HTLV-III.

7. The method of Claim 4, further comprising selecting an amount of virus for assay such that 50 to 80% of the H9 cells are infected.

8. A method for the *in vitro* assay of the protective effects induced by neutralizing HTLV-III antibodies on HTLV-III virus by addition of sera containing neutralizing antibodies, comprising measuring residual viral infectivity by addition of a monoclonal antibody specific for HTLV or p24 HTLV.

9. The method according to Claim 8, further comprising a hold period of three days prior to the addition of the monoclonal antibodies to allow for viral infection.

10. The method according to Claim 8, wherein an immune fluorescent assay is used for reaction of an antigen and the monoclonal antibody.

**Patentansprüche**

1. Verfahren zur In-Vitro-Messung der Neutralisation der HTLV-III-Virus-Infektivität, das umfaßt:
a) Behandeln des genannten Virus mit natürlichen menschlichen Antikörpern in Seren, die Antikörper für HTLV-III enthalten, und
b) Testen der restlichen viralen Infektivität zur Bestimmung des Maßes des durch die natürlichen Antikörper gegebenen Schutzes.

2. Verfahren nach Anspruch 1, wobei der Test mit dem für HTLV-III spezifischen monoklonalen Antikörper ausgeführt wird.

3. Verfahren nach Anspruch 2, wobei der monoklonale Antikörper Anti-HTLV-III p24 ist.

4. Verfahren zur In-Vitro-Messung der Neutralisation der HTLV-III-Virus-Infektivität, das umfaßt:
a) Behandeln des genannten Virus mit Seren, die natürliche menschliche Antikörper enthalten,
b) Infizieren und Inkubieren der sich ergebenden Kultur mit H9-Zellen, und
c) Testen der restlichen Infektivität mit einem für HTLV-III oder HTLV-III p24 spezifischen monoklonalen Antikörper.

5. Verfahren nach Anspruch 4, wobei der monoklonale Antikörper spezifisch für HTLV-III p24 ist.

6. Verfahren nach Anspruch 4, wobei der monoklonale Antikörper für HTLV-III spezifische ist.

7. Verfahren nach Anspruch 4, welches das Auswählen einer Virusmenge zum Test derart umfaßt, daß 50% bis 80% der H9-Zellen infiziert werden.

8. Verfahren zum In-Vitro-Testen der Schutzwirkungen, die durch Neutralisieren von HTLV-III-Antikörpern auf HTLV-III-Virus durch Zugabe von neutralisierenden Antikörpern enthaltenden Seren induziert werden, wobei die restliche virale Infektivität durch Zugabe eines monoklonalen Antikörpers gemessen wird, der für HTLV oder p24-HTLV spezifisch ist.

9. Verfahren nach Anspruch 8, das weiter eine Halteperiode von drei Tagen vor der Zugabe der monoklonalen Antikörper umfaßt, um eine virale Infektion zu ermöglichen.

10. Verfahren nach Anspruch 8, wobei ein Immunfluoreszenztest zur Reaktion eines Antigens und des monoklonalen Antikörpers verwendet wird.

**Revendications**

1. Méthode pour la mesure, *in vitro*, de la neutralisation de l'inefectivité du virus HTLV-III comprenant:

EP 0 193 284 B1

(a) le traitement dudit virus avec des anticorps humains naturels dans des sérums contenant de l'anticorps au HTLV-III; et

(b) la détermination de l'infectivité virale résiduelle pour apprécier la mesure de protection fournie par les anticorps naturels.

2. Méthode selon la revendication 1, dans lequelle la détermination est effectuée par l'anticorps monoclonal spécifique pour HTLV-III.

3. Méthode selon la revendication 2, dans laquelle l'anticorps monoclonal est l'anti-HTLV-III p24.

4. Méthode pour la mesure, *in vitro*, de la neutralisation de l'infectivité du virus HTLV-III comprenant:

(a) le traitement dudit virus avec des sérums contenant des anticorps humains naturels;

(b) l'infection et l'incubation de la culture qui résulte avec des cellules H9; et

(c) la détermination de l'infectivité résiduelle avec un anticorps monoclonal qui est spécifique pour le HTLV-III ou pour le HTLV-III p24.

5. Méthode selon la revendication 4, dans laquelle l'anticorps monoclonal est spécifique pour le HTLV-III p24.

6. Méthode selon la revendication 4, dans laquelle l'anticorps monoclonal est spécifique pour le HTLV-III.

7. Méthode selon la revendication 4, qui comprend également la sélection d'une quantité de virus pour la détermination telle que 50 à 80% des cellules H9 soient infectées.

8. Méthode pour la détermination *in vitro* des effets protecteurs déclenchés par la neutralisation des anticorps HTLV-III sur le virus HTLV-III par l'additional des sérums contenant des anticorps neutralisants, comprenant la mesure de l'infectivité virale résiduelle par l'addition d'un anticorps monoclonal spécifique pour HTLV ou p24 HTLV.

9. Méthode selon la revendication 8, qui comprend également une période de retardement de trois jours avant l'additional des anticorps monoclonaux afin de permettre l'infection virale.

10. Méthode selon la revendication 8, dans laquelle on utilise, pour la réaction d'un antigène et de l'anticorps monoclonal, une détermination par immunofluorescence.

EP 0 193 284 B1